# EUROPEAN PATENT APPLICATION

(11) **EP 4 809 945 A1**
(43) Date of publication of application: **23.09.2026**
(21) Application number: 24949290.1
(22) Date of filing: 22.08.2024
(51) Int. Cl.: A61B 17/3207, A61B 17/3205, A61B 17/221, A61B 17/22

(54) **THROMBECTOMY APPARATUS**

(30) Priority: 02.08.2024 CN 202411056326
(71) Applicant: Shenzhen Betterway Medtech Co., Ltd., Shenzhen, Guangdong 518000 (CN)
(72) Inventor: WU, Xingyu, Shenzhen, Guangdong 518000 (CN); LI, Zhenjie, Shenzhen, Guangdong 518000 (CN); LONG, Han, Shenzhen, Guangdong 518000 (CN)
(74) Representative: Bayramoglu et al.
(86) International application number: PCT/CN2024/113879
(87) International publication number: WO 2026/025565

(57) **Abstract**

A thrombectomy apparatus (1), comprising a retrieval frame (10), a pulling tube (20), and an inner tube (60). The retrieval frame (10) comprises a proximal support (A) and a distal support (B) connected to the far end of the proximal support (A). The retrieval frame (10) is provided with an inner cavity penetrating through the proximal support (A) and the distal support (B). The proximal support (A) comprises a first axial section (Al) and a second axial section (A2) connected to the far end of the first axial section (Al). An oblique opening part (11) in communication with the inner cavity is formed at the near end of the first axial section (Al), such that the first axial section (Al) has a tip structure pointing to the near end. The second axial section (A2) has a circumferential outer contour. The range of the axial tensile strength ratio of the first axial section (Al) to the second axial section (A2) is [0.6, 1]. The pulling tube (20) is connected to the tip structure. The inner tube (60) slidably passes through the pulling tube (20), and the far end of the inner tube (60) is connected to the far end of the distal support (B). The inner tube (60) slides to the far end relative to the pulling tube (20), so that the retrieval frame (10) can be radially contracted. The thrombectomy apparatus (1) is less irritating to blood vessels during thrombectomy and exhibits relatively good wall apposition at the open end.

## Description

### TECHNICAL FIELD

The present invention relates to the technical field of medical devices, and in particular to a thrombectomy apparatus.

### BACKGROUND

This section merely provides background information related to the present disclosure and does not necessarily constitute prior art.

At present, treatment regimens for deep venous thrombosis (DVT) mainly include a mechanical thrombectomy, a pharmacological thrombolysis, and a negative pressure aspiration. Among them, the mechanical thrombectomy generally refers to scraping and collecting the thrombi on the blood vessel wall with a thrombectomy stent, and then removing the thrombi out of the body.

During thrombectomy, the inner diameter of the deep vein gradually decreases along the moving direction of the thrombectomy stent. The thrombectomy stent is radially compressed by the blood vessel, and in turn, the thrombectomy stent radially abuts against the blood vessel wall. Therefore, when the thrombectomy stent moves in a blood vessel with a small inner diameter, the thrombectomy stent causes relatively high irritation to the blood vessel. Thus, to reduce irritation to the blood vessel and avoid blood vessel injury, it is desirable that the radial dimension of the thrombectomy stent can be reduced before entering a small blood vessel, especially the portion with high radial support performance, which causes the greatest irritation to the blood vessel wall, so reducing the radial dimension of this portion can reduce irritation to the blood vessel to a greater extent. However, when the radial dimension of the portion with high radial support performance is reduced, the portion with low radial support performance shrinks to a greater extent. For example, the oblique opening portion at the proximal end shrinks to a greater extent radially, resulting in failure of the oblique opening portion to conform to the shape of the blood vessel wall, thereby failing to effectively scrape thrombi attached to the blood vessel wall.

### SUMMARY

Based on the above, it is necessary to provide a thrombectomy apparatus that causes less irritation to blood vessels and has good apposition at the opening end during thrombectomy.

A thrombectomy apparatus, including:
a retrieval frame, including a proximal support and a distal support connected to a distal end of the proximal support, the retrieval frame having an inner cavity penetrating the proximal support and the distal support, the proximal support including a first axial section and a second axial section connected to a distal end of the first axial section, an oblique opening portion communicating with the inner cavity being provided at a proximal end of the first axial section, the first axial section having a tip structure pointing to the proximal end, the second axial section having a circumferential outer contour, and a ratio of axial tensile strength of the first axial section to that of the second axial section being ranged from 0.6 to 1;
a pulling tube, connected to the tip structure; and
an inner tube, slidably disposed through the pulling tube, a distal end of the inner tube being connected to a distal end of the distal support, and the retrieval frame being radially shrinkable in response to a sliding of the inner tube distally relative to the pulling tube.

When the above thrombectomy apparatus is used for thrombectomy, the oblique opening portion is first positioned at the distal end of the thrombus, and then a proximal pulling force is applied to the oblique opening portion via the pulling tube, so as to drive the retrieval frame to move proximally in the blood vessel. During this process, thrombi on the inner wall of the blood vessel can be scraped off by the oblique opening portion and collected in the inner cavity of the retrieval frame. In an embodiment, a plurality of busbar limit slots are defined on a first side of the first fixed plate, and each of the plurality of busbar limit slots is configured to position the busbar.

During thrombectomy, before the retrieval frame enters a portion with a small inner diameter from a portion with a large inner diameter of the blood vessel, the inner tube is slid distally relative to the pulling tube to radially contract the retrieval frame. This prevents the retrieval frame from being excessively compressed by the blood vessel after entering the portion with a small inner diameter of the blood vessel with a large radial contour and then reversely irritating the blood vessel wall, helping reduce irritation to the blood vessel.

After the retrieval frame enters the portion with a small inner diameter of the blood vessel, the pulling tube is pulled proximally to move the retrieval frame proximally. During movement, the tip structure penetrates the thrombus, and the oblique opening portion can scrape thrombi on the blood vessel wall. Since the tip structure penetrates the thrombus with relatively low resistance, the retrieval frame can be pulled to move by pulling the pulling tube with a small force, thereby helping avoid severe scraping between the second axial section with a circumferential outer contour and the blood vessel wall caused by pulling the retrieval frame with an excessive force, and further helping reduce irritation to the blood vessel wall.

Furthermore, since the ratio of axial tensile strength of the first axial section to that of the second axial section is ranged from 0.6 to 1, when the retrieval frame is pulled by the pulling tube, the radial contraction amplitudes of the first axial section and the second axial section are closer to each other. This helps prevent the first axial section from contracting radially excessively to cause poor apposition or non- apposition of the oblique opening portion, leading to thrombectomy failure, when the second axial section contracts radially to a degree that causes low irritation to the blood vessel wall.

Therefore, during thrombectomy, the above thrombectomy apparatus causes less irritation to the blood vessel wall and has good apposition, enabling efficient thrombectomy while helping avoid secondary injury to the patient.

### BRIEF DESCRIPTION OF THE DRAWINGS

To clearly illustrate the technical solutions in the embodiments of the present invention, the drawings required for describing the embodiments will be briefly introduced below. Obviously, the drawings described below are merely some embodiments of the present invention, and those skilled in the art can obtain other drawings based on these drawings without creative efforts.

Wherein:
FIG. 1 is a structural schematic view of a thrombectomy apparatus in an embodiment;
FIG. 2 is a top view of FIG. 1;
FIG. 3 is a planar expanded view of the proximal support in the embodiment shown in FIG. 1, taking a straight line, as a cutting line, from a distal end of the oblique opening portion to a distal end of the distal support;
FIG. 4 is an enlarged view at X in FIG. 3;
FIG. 5A is a planar expanded view of a proximal support in another embodiment, taking a straight line, as a cutting line, from a distal end of the oblique opening portion to a distal end of the distal support;
FIG. 5B is an enlarged view at Y in FIG. 5A;
FIG. 6 is a partial structural schematic view of a proximal support in another embodiment in planar expansion, taking a straight line, as a cutting line, from a distal end of the oblique opening portion to a distal end of the distal support; and
FIG. 7 is a planar expanded view of a proximal support in another embodiment, taking a straight line, as a cutting line, from a distal end of the oblique opening portion to a distal end of the distal support.

### DETAILED DESCRIPTION OF THE EMBODIMENTS

The technical solutions in the embodiments of the present invention will be clearly and completely described below with reference to the accompanying drawings in the embodiments of the present invention. Obviously, the described embodiments are merely part of the embodiments of the present invention, not all of them. All other embodiments obtained by those skilled in the art based on the embodiments of the present invention without creative efforts shall fall within the protection scope of the present invention.

In the description of the embodiments of the present invention, it should be noted that orientation or positional relationships indicated by terms such as "center", "upper", "lower", "left", "right", "vertical", "horizontal", "inner" and "outer" are based on the orientation or positional relationships shown in the drawings, which are merely for convenience of describing the embodiments of the present invention and simplifying the description, and do not indicate or imply that the referred device or element must have a specific orientation, be constructed and operated in a specific orientation, and thus shall not be construed as limiting the embodiments of the present invention. In addition, terms "first", "second" and "third" are for descriptive purposes only and shall not be construed as indicating or implying relative importance.

In the description of the embodiments of the present invention, it should be noted that unless otherwise explicitly specified and limited, terms "mounted", "connected" and "linked" shall be understood broadly. For example, they may be fixed connections, replaceable connections or integral connections; they may be mechanical connections or electrical connections; they may be direct connections or indirect connections via an intermediate medium; they may be internal communication between two elements. Those skilled in the art can understand the specific meanings of the above terms in the embodiments of the present invention according to specific situations.

In the field of interventional medical devices, the end of a medical device implanted into a human or animal body that is closer to an operator is generally referred to as the "proximal end", and the end farther from the operator is referred to as the "distal end". The "proximal end" and "distal end" of any component of the medical device are defined according to this principle. "Axial" generally refers to the length direction of the medical device during delivery, and "radial" generally refers to a direction of the medical device that is not parallel to the "axial direction". The "axial" and "radial" of any component of the medical device are defined according to this principle. "Circumferential" refers to the circumferential direction, i.e., the direction around the axis of a lumen structure or cylinder.

### First Embodiment

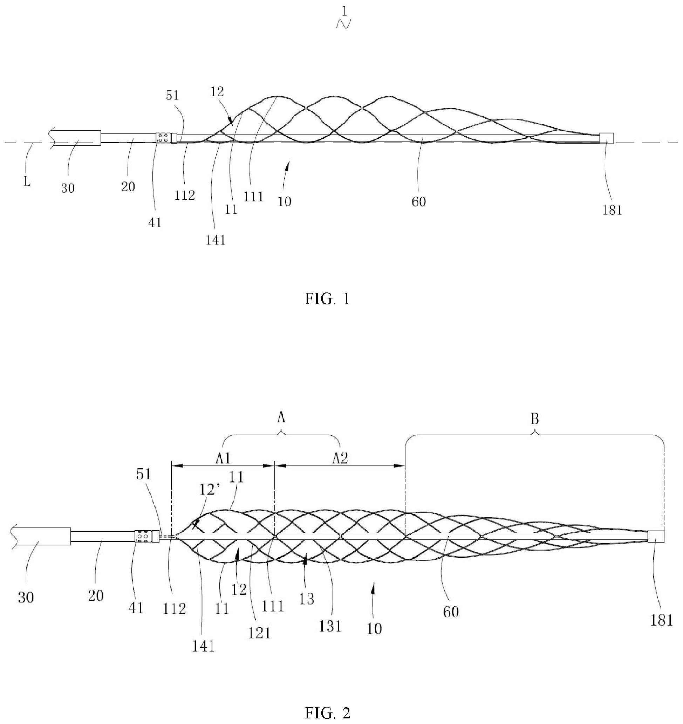

Referring to FIGS. 1 and 2, the present disclosure provides a thrombectomy apparatus 1, which can be used to remove thrombi in a blood vessel of a patient. The thrombectomy apparatus 1 includes a retrieval frame 10, a pulling tube 20 and a delivery sheath 30, where a distal end of the pulling tube 20 is connected to a proximal end of the retrieval frame 10, and the delivery sheath 30 is slidably sleeved on the pulling tube 20. The delivery sheath 30 is a tubular structure and can slide axially along the pulling tube 20 to load and release the retrieval frame 10.

The retrieval frame 10 is a mesh structure with an inner cavity. The retrieval frame 10 can be formed by weaving and shaping weaving wires or by cutting and shaping a hollow tube. The weaving wires can be nickel- titanium alloy wires, stainless steel wires or other metal wires. The hollow tube can be a metal tube such as a nickel- titanium alloy tube or a stainless steel tube, or a polymer tube.

The retrieval frame 10 is radially shrinkable. The retrieval frame 10 can be compressed to a loading size under a binding force (radial compression force on the retrieval frame 10 or axial tensile force applied to both ends of the retrieval frame 10), so that the retrieval frame 10 can be loaded into the delivery sheath 30 and delivered in the blood vessel of the patient via the delivery sheath 30. After reaching a desired position, the delivery sheath 30 is operated to move proximally relative to the retrieval frame 10 and the pulling tube 20 to expose the retrieval frame 10.

The retrieval frame 10 is self- expandable. When the binding force acting on the retrieval frame 10 is removed, the retrieval frame 10 can expand to a radially expanded state. For example, after the delivery sheath 30 sleeved on the retrieval frame 10 is retracted to be separated from the retrieval frame 10, the retrieval frame 10 can radially self- expand to a radially expanded state.

Referring to FIG. 2, the retrieval frame 10 axially includes a proximal support A and a distal support B connected to each other. The proximal support A is located at the proximal end of the distal support B, and both the proximal support A and the distal support B are part of the mesh structure. The proximal support A and the distal support B are connected and together form the inner cavity of the retrieval frame 10. The inner cavity of the retrieval frame 10 penetrates the proximal support A and the distal support B. An oblique opening portion 11 communicating with the inner cavity is provided at the proximal end of the proximal support A. The oblique opening portion 11 is a circumferentially closed structure and can scrape thrombi when moving in a blood vessel under an external force.

With continued reference to FIG. 2, the proximal support A includes a first axial section A1 and a second axial section A2 connected in sequence from proximal to distal. An oblique opening portion 11 communicating with the inner cavity is formed at the proximal end of the first axial section A1, so that the first axial section A1 has a tip structure pointing to the proximal end. The axial length of the oblique opening portion 11 is equal to that of the first axial section A1. A proximal end of the second axial section A2 is connected to the first axial section A1, and the second axial section A2 has a circumferential outer contour. The ratio of axial tensile strength of the first axial section A1 to that of the second axial section A2 is ranged from 0.6 to 1, so that when axial tensile force is applied to both ends of the retrieval frame 10, the ratio of axial stretched lengths of the first axial section A1 to the second axial section A2 is ranged from 0.6 to 1.

The ratio of axial tensile strength of the first axial section A1 to that of the second axial section A2 can be measured by the following method: applying axial tensile force to both ends of the retrieval frame 10 to axially stretch both the first axial section A1 and the second axial section A2 and cause radial contraction deformation. While maintaining the force applied to the retrieval frame 10, record the axial lengths of the axially stretched first axial section A1 and second axial section A2, and then calculate the ratio of the axial length of the first axial section A1 to that of the second axial section A2, which is the ratio of axial tensile strength of the first axial section A1 to that of the second axial section A2.

With continued reference to FIGS. 1 and 2, the oblique opening portion 11 includes a distal end 111 and a proximal end 112, and an axial distance between the distal end 111 and the proximal end 112 is greater than zero. The proximal end 112 of the oblique opening portion 11 is a tip structure. The oblique opening portion 11 is a circumferentially closed structure and can scrape thrombi circumferentially when moving in a blood vessel under an external force. When the retrieval frame 10 is located in a blood vessel and the binding force acting on the retrieval frame 10 is removed, the retrieval frame 10 can self- expand to a radially expanded state, so that the oblique opening portion 11 is apposed to the inner wall of the blood vessel.

Referring to FIGS. 1 and 2, the thrombectomy apparatus 1 further includes a collar 41 connected to the proximal end 112 of the oblique opening portion 11 via a connecting strip 51.

The pulling tube 20 is a hollow tubular structure. A distal end of the pulling tube 20 is connected to the collar 41, so as to be connected to the proximal end 112 of the oblique opening portion 11 via the collar 41 and the connecting strip 51. This allows the pulling tube 20 to apply force to the retrieval frame 10, so as to push the retrieval frame 10 to the distal end of the thrombus during thrombectomy, and apply a pulling force to the retrieval frame 10 to move the retrieval frame 10 from distal to proximal in the blood vessel to scrape thrombi after the retrieval frame 10 is delivered to a desired position (e.g., at the distal end of the thrombus).

Referring to FIGS. 1 and 2, the thrombectomy apparatus 1 further includes an inner tube 60 slidably disposed through the pulling tube 20. A distal end of the inner tube 60 is connected to a distal end of the distal support B, and the outer contour of the retrieval frame 10 can be radially reduced when the inner tube 60 slides distally relative to the pulling tube 20.

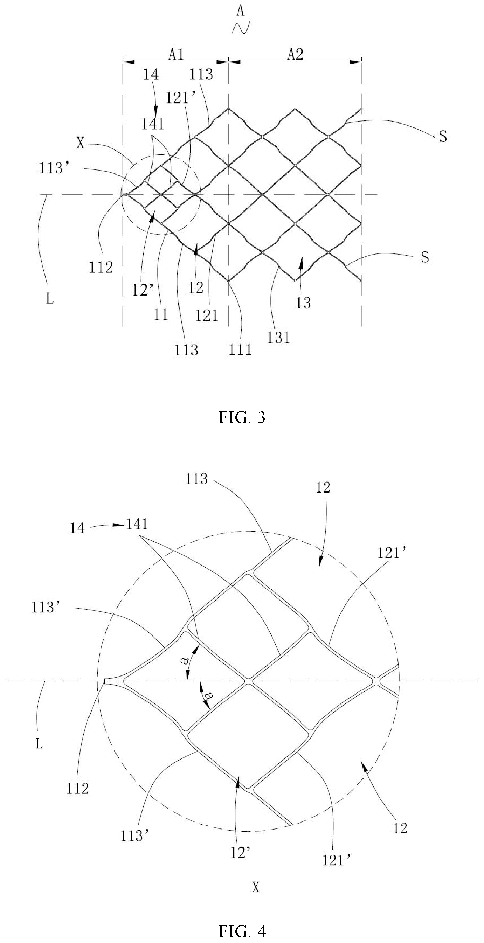

Referring to FIG. 3, when the retrieval frame 10 is planarly expanded by taking a straight line, as a cutting line, from the distal end 111 of the oblique opening portion 11 to the distal end 181 of the distal support B, the first axial section A1 is a first mesh structure with a triangular outer contour. The proximal end 112 of the oblique opening portion 11 is a vertex of the triangle, and two edges 113 extending distally from the vertex form the oblique opening portion 11 when closed. The first mesh structure includes a plurality of first meshes 12 defined by the two edges 113 and a plurality of first mesh rods 121.

The tip structure of the first axial section A1 points to the proximal end, and in the above planar expanded state, the first axial section A1 is a first mesh structure defined by two edges 113 and a plurality of first mesh rods 121, so that during thrombectomy, the first axial section A1 can better overcome thrombus resistance and smoothly scrape thrombi. In addition, the first axial section A1 with this structure has certain structural stability, helping avoid deformation that leads to failure to effectively scrape thrombi.

In an embodiment, the ratio of axial tensile strength of the first axial section A1 to that of the second axial section A2 is ranged from 0.6 to 1.

When the thrombectomy apparatus 1 is used for thrombectomy, the oblique opening portion 11 of the retrieval frame 10 is first positioned at the distal end of the thrombus, then the binding force acting on the retrieval frame 10 is removed, and the retrieval frame 10 self- expands so that the oblique opening portion 11 is apposed to the blood vessel wall. Since the oblique opening portion 11 is a circumferentially closed structure, the oblique opening portion 11 can be apposed to the blood vessel wall in a 360° circumferential direction. A pulling force is applied to the pulling tube 20 to drive the retrieval frame 10 to move proximally, so as to cut thrombi on the blood vessel wall. Thrombi cut off by the oblique opening portion 11 enter the inner cavity of the retrieval frame 10 through the oblique opening portion 11 to be captured and thereby carried out of the body by the retrieval frame 10.

During thrombectomy, before the retrieval frame 10 enters a portion with a small inner diameter from a portion with a large inner diameter, a distal pushing force is applied to the inner tube 60 to slide the inner tube 60 distally relative to the pulling tube 20 to radially contract the retrieval frame 10. This prevents the retrieval frame 10 from being excessively compressed by the blood vessel after entering the portion with a small inner diameter of the blood vessel with a large radial contour and then reversely irritating the blood vessel, helping reduce irritation to the blood vessel.

When the retrieval frame 10 is subjected to proximal pulling force to perform thrombectomy in a blood vessel with a small inner diameter, since the tip structure penetrates the thrombus with relatively low resistance, this helps avoid severe scraping between the second axial section A2 with a circumferential outer contour and the blood vessel wall caused by pulling the retrieval frame 10 with an excessive force, and further helps reduce irritation to the blood vessel wall.

Furthermore, when the retrieval frame 10 is subjected to axial tensile force, since the ratio of axial tensile strength of the first axial section A1 to that of the second axial section A2 is ranged from 0.6 to 1, the difference in axial tensile strength between the first axial section A1 and the second axial section A2 is small, so the radial contraction amplitudes of the first axial section A1 and the second axial section A2 are closer to each other. This helps prevent the first axial section A1 from contracting radially excessively to cause poor apposition or non- apposition of the oblique opening portion 11, leading to thrombectomy failure, when the second axial section A2 contracts radially to a degree that causes low irritation to the blood vessel wall.

Referring to FIG. 2, the second axial section A2 is cylindrical and has a cylindrical inner cavity open at both ends. A distal end of the second axial section A2 is connected to a proximal end of the distal support B. An end of the second axial section A2 away from the distal support B is connected to the first axial section A1. The second axial section A2 is cylindrical and has a circumferential outer contour, so that during thrombectomy, the second axial section A2 can well maintain a state of apposition to the blood vessel wall, helping avoid thrombus escape and thereby improving thrombectomy efficiency.

With continued reference to FIG. 2, the distal support B is tapered, a small- diameter end of the distal support B is located at the distal end, a large- diameter end of the distal support B is located at the proximal end, and the large- diameter proximal end of the distal support B is connected to the distal end of the second axial section A2. The diameter of portions of the distal support B other than the portion connected to the proximal support A is smaller than that of the proximal support A, so the distal support B causes less irritation to the blood vessel during thrombectomy. The distal support B has a tapered inner cavity, a large- diameter end of the distal support B is an open end, and a small- diameter end of the distal support B is a closed end. The closed end is a closed end of the retrieval frame 10.

Referring to FIG. 3, when the retrieval frame 10 is planarly expanded by taking a straight line, as a cutting line, from the oblique opening portion 11 and the distal end 181 of the distal support B, the second axial section A2 is a second mesh structure defined by two radially opposite wavy lines S. Proximal ends of the two wavy lines S are respectively connected to distal ends of the two edges 113, a maximum radial distance between the two opposite wavy lines S is equal to a radial distance between the distal ends of the two edges 113, and the second mesh structure includes a plurality of second meshes 13 defined by a plurality of second mesh rods 131.

A ratio of a total volume of the two edges 113 and the plurality of first mesh rods 121 of the first axial section A1 to a total volume of the plurality of second mesh rods 131 of the second axial section A2 is ranged from 0.6 to 1, so that the ratio of axial tensile strength of the first axial section A1 to that of the second axial section A2 is ranged from 0.6 to 1. Further, when both ends of the retrieval frame 10 are pulled, the radial contraction amplitudes of the first axial section A1 and the second axial section A2 are closer to each other, so that when the retrieval frame 10 performs thrombectomy in a portion with a small inner diameter of a blood vessel, irritation of the second axial section A2 to the inner wall of the blood vessel is reduced, and the oblique opening portion 11 can still be kept apposed to the inner wall of the blood vessel.

Referring to FIG. 3, in an embodiment, a reinforcement structure 14 is arranged in the first mesh structure to increase the axial tensile strength of the first axial section A1. In an embodiment, a ratio of a total volume of the two edges 113, the plurality of first mesh rods 121 and the reinforcement structure 14 of the first axial section A1 to a total volume of the plurality of second mesh rods of the second axial section A2 is ranged from 0.65 to 1, so that the ratio of axial tensile strength of the first axial section A1 to that of the second axial section A2 is ranged from 0.65 to 1. When both ends of the retrieval frame 10 are axially stretched to radially contract, the radial contraction amplitudes of the first axial section A1 and the second axial section A2 are closer to each other, helping prevent the first axial section A1 from contracting radially excessively to cause poor apposition or non-apposition of the oblique opening portion 11, leading to thrombectomy failure, when the second axial section A2 contracts radially to a degree that causes low irritation to the blood vessel wall.

Referring to FIG. 3, the plurality of first meshes 12 include a most proximal mesh, marked as 12' to distinguish it from other first meshes 12. Referring also to FIG. 4, in an embodiment, the most proximal mesh 12' is a quadrilateral mesh defined by parts of the two edges 113 and two first mesh rods 121, and a proximal vertex of the most proximal mesh 12' is a proximal vertex of the first mesh structure. For convenience of description, a part of each edge 113 for defining the most proximal mesh 12' is named a proximal mesh rod, and a first mesh rod for defining the most proximal mesh 12' is named a distal mesh rod. To distinguish the proximal mesh rods and distal mesh rods from other parts of the edges 113 and other first mesh rods 121, the proximal mesh rods are specially marked as 113', and the distal mesh rods are specially marked as 121'.

With continued reference to FIGS. 3 and 4, the reinforcement structure 14 includes at least one reinforcement rod 141 arranged in the most proximal mesh 12'. The reinforcement rod 141 is a rod- shaped structure with certain rigidity. In the embodiment shown in FIGS. 3 and 4, the number of the reinforcement rods 141 is two. Two ends of each reinforcement rod 141 are respectively fixedly connected to two of the two edges 113 and the two first mesh rods 121. In this embodiment, two ends of each reinforcement rod 141 are respectively fixedly connected to one proximal mesh rod 113' and one distal mesh rod 121', the two reinforcement rods 141 intersect each other, and the two reinforcement rods 141 are fixedly connected at the intersection.

When the retrieval frame 10 is planarly expanded by taking a straight line, as a cutting line, from the distal end 111 of the oblique opening portion 11 to the distal end 181 of the distal support B, each reinforcement rod 141 forms an included angle α with a straight line L passing through the proximal end 112 of the oblique opening portion 11 and the distal end 181 of the distal support B, and the included angle α is ranged from 0° to 90°. In an embodiment, the most proximal mesh 12' has a diagonal line along the axial direction, and the diagonal line coincides with the straight line L.

When the included angle α is ranged from 0° to 12°, the length of the reinforcement rod 141 is greater than or equal to 1/2 of the axial length of the most proximal mesh 12'.

When the included angle α is ranged from 12° to 74°, a minimum distance from a connection point of each reinforcement rod 141 with two of the two first mesh rods 121 and two edges 113 to ends of the two first mesh rods 121 and two edges 113 is greater than or equal to 1/5 of the length of the corresponding first mesh rod 121 or edge 113. It should be noted that "a minimum distance from a connection point of each reinforcement rod 141 with two of the two first mesh rods 121 and two edges 113 to ends of the two first mesh rods 121 and two edges 113" refers to a minimum distance from a connection point of each reinforcement rod 141 with two of the two distal mesh rods 121' and two proximal mesh rods 113' to ends of the two distal mesh rods 121' and two proximal mesh rods 113'; and "greater than or equal to 1/5 of the length of the corresponding first mesh rod 121 or edge 113" refers to greater than or equal to 1/5 of the length of the corresponding distal mesh rod 121' or proximal mesh rod 113'.

When the included angle α is ranged from 74° to 90°, a minimum distance from a connection point of each reinforcement rod 141 with two of the two first mesh rods 121 and two edges 113 to ends of the two first mesh rods 121 and two edges 113 is greater than or equal to 1/5 of the length of the corresponding first mesh rod 121 or edge 113, and the length of the reinforcement rod 141 is greater than or equal to 1/2 of the width of the most proximal mesh 12'. That is, a minimum distance from a connection point of each reinforcement rod 141 with two of the two distal mesh rods 121' and two proximal mesh rods 113' to ends of the two distal mesh rods 121' and two proximal mesh rods 113' is greater than or equal to 1/5 of the length of the corresponding distal mesh rod 121' or proximal mesh rod 113', and the length of the reinforcement rod 141 is greater than or equal to 1/2 of the width of the most proximal mesh 12'.

In this embodiment, a ratio of a total volume of the two edges 113 (including proximal mesh rods 113'), the plurality of first mesh rods 121 (including distal mesh rods 121') and the reinforcement rods 141 of the first axial section A1 to a total volume of the plurality of second mesh rods 131 of the second axial section A2 is ranged from 0.65 to 1, so that the ratio of axial tensile strength of the first axial section A1 to that of the second axial section A2 is ranged from 0.65 to 1, thereby further reducing the difference in axial tensile strength between the first axial section A1 and the second axial section A2. Before entering a portion with a small inner diameter of a blood vessel, axial tensile force is applied to both ends of the retrieval frame 10 via the pulling tube 20 and the inner tube 60 to reduce the radial dimension of the retrieval frame 10. The small difference in axial tensile strength between the first axial section A1 and the second axial section A2 allows the radial reduction amplitudes of the first axial section A1 and the second axial section A2 to be closer to each other, helping prevent the first axial section A1 from contracting radially excessively to cause poor apposition or non-apposition of the oblique opening portion 11, leading to thrombectomy failure, when the second axial section A2 contracts radially to a degree that causes low irritation to the blood vessel wall.

The axial length of the most proximal mesh 12' refers to the maximum length of the most proximal mesh 12' in the axial direction of the retrieval frame 10. The width of the most proximal mesh 12' refers to the maximum dimension of the most proximal mesh 12' in a direction perpendicular to the axial direction when the retrieval frame 10 is planarly expanded.

In other embodiments, the number of the above reinforcement rods 141 can be one or more. When the number of the reinforcement rods 141 in the most proximal mesh 12' is multiple, the reinforcement rods 141 can be parallel to or intersect each other. When the two reinforcement rods 141 intersect, the two intersecting reinforcement rods 141 are fixedly connected at the intersection. In addition, when the number of reinforcement rods 141 in the most proximal mesh 12' is multiple, the included angles α formed by different reinforcement rods 141 with the straight line L can be in the same range or different ranges among the above three angle ranges (i.e., 0° to 12°, 12° to 74°, and 74° to 90°).

During applying a pulling force to the pulling tube 20 of this embodiment to drive the retrieval frame 10 to move proximally, when the first meshes 12 of the proximal support A are stretched to become longer or tend to become longer, since the reinforcement rods 141 have certain rigidity and are located in the first axial section A1, and two ends of each reinforcement rod 141 are respectively fixedly connected to two mesh rods of the most proximal mesh 12' in the first axial section A1 (specifically, any two of the two proximal mesh rods 113' and two distal mesh rods 121'), the reinforcement rods 141 can provide a binding force to the most proximal mesh 12'. Therefore, arranging the reinforcement rods 141 in the most proximal mesh 12' can reduce the amplitude of axial stretching of the most proximal mesh 12' or prevent axial stretching. In addition, since the most proximal mesh 12' is a direct stress part, the arrangement of the reinforcement rods 141 resists part of the tensile force, so that the tensile force transmitted to other first meshes 12 of the first axial section A1 is weakened, slowing or preventing axial stretching of other meshes 12 of the first axial section A1. That is, the arrangement of the reinforcement rods 141 increases the axial tensile strength of the first axial section A1. Thus, the reinforcement rods 141 can reduce the amplitude of axial stretching of the first axial section A1 during driving the retrieval frame 10 to move in the blood vessel, i.e., reduce the amplitude of axial stretching of the oblique opening portion 11, thereby reducing the amplitude of radial inward contraction of the oblique opening portion 11 caused by axial stretching, so as to improve the apposition of the oblique opening portion 11. In addition, since the arrangement of the reinforcement rods 141 increases the axial tensile strength of the first axial section A1, the axial tensile strength of the first axial section A1 is closer to that of the second axial section A2. Before the first axial section A1 and the second axial section A2 enter a portion with a small inner diameter of a blood vessel, when axial tensile force is applied to the retrieval frame 10 via the pulling tube 20 and the inner tube 60 to radially contract the retrieval frame 10, the radial contraction amplitudes of the first axial section A1 and the second axial section A2 are closer to each other, so that irritation of the second axial section A2 to the blood vessel can be reduced while the oblique opening portion 11 can be kept apposed to the inner wall of the blood vessel.

When the proximal support A is planarly expanded, the reinforcement rod 141 forms an included angle α with the straight line L, and the included angle α is ranged from 0° to 90°. When the included angle α is ranged from 0° to 12°, the length of the reinforcement rod 141 is greater than or equal to 1/2 of the axial length of the most proximal mesh 12', so that the reinforcement rod 141 has sufficient length and thus sufficient support performance, thereby increasing the axial tensile strength of the first axial section A1. In addition, when the included angle α is ranged from 0° to 12° and the length of the reinforcement rod 141 is greater than or equal to 1/2 of the axial length of the most proximal mesh 12', the reinforcement rod 141 can provide a binding force to a large area of the most proximal mesh 12' to increase the axial tensile strength of the most proximal mesh 12', so as to prevent reduced apposition of the oblique opening portion 11 caused by excessive stretching during thrombectomy. In addition, the reinforcement rod 141 can provide a binding force to a large area of the most proximal mesh 12' to increase the axial tensile strength of the most proximal mesh 12', thereby increasing the axial tensile strength of the first axial section A1, reducing the difference in axial tensile strength between the first axial section A1 and the second axial section A2, making the radial contraction amplitudes of the first axial section A1 and the second axial section A2 closer to each other when reducing the radial dimension of the retrieval frame 10, and helping prevent the first axial section A1 from contracting radially excessively to cause poor apposition or non-apposition of the oblique opening portion 11, leading to thrombectomy failure, when the second axial section A2 contracts radially to a degree that causes low irritation to the blood vessel wall during thrombectomy in a blood vessel with a small inner diameter. In contrast, when the included angle α is within this range but the length of the reinforcement rod 141 is small (e.g., less than 1/2 of the axial length of the most proximal mesh 12'), for example, α=0° and the length of the reinforcement rod 141 is equal to 1/4 of the axial length of the most proximal mesh 12', the length of the reinforcement rod 141 is small, resulting in low support effect of the reinforcement rod 141 on the most proximal mesh 12'. The reinforcement rod 141 can only provide a binding force to a small area of the most proximal mesh 12', i.e., has little effect on reducing the difference in axial tensile strength between the first axial section A1 and the second axial section A2. When the oblique opening portion 11 is subjected to pulling force during thrombectomy, it has little effect on slowing excessive stretching of the oblique opening portion 11 to slow the degree of separation from the inner wall of the blood vessel, and also has little effect on reducing the difference in axial tensile strength between the first axial section A1 and the second axial section A2.

Similarly, when the included angle α is ranged from 12° to 74°, a minimum distance from a connection point of each reinforcement rod 141 with a mesh rod (e.g., proximal mesh rod 113' or distal mesh rod 121') to an end of the mesh rod is greater than or equal to 1/5 of the length of the mesh rod, so that the reinforcement rod has a long length and can provide a binding force to a large area of the most proximal mesh 12', so as to prevent reduced apposition of the oblique opening portion 11 caused by excessive stretching during thrombectomy. In addition, the reinforcement rod 141 can provide a binding force to a large area of the most proximal mesh 12' to reduce the difference in axial tensile strength between the first axial section A1 and the second axial section A2, so that when reducing the radial dimension of the retrieval frame 10, the reduction amplitudes of the first axial section A1 and the second axial section A2 are closer to each other. The retrieval frame 10 can reduce irritation of the second axial section A2 to the blood vessel while keeping the oblique opening portion 11 apposed to the inner wall of the blood vessel. Similarly, when the included angle α is ranged from 74° to 90°, a minimum distance from a connection point of each reinforcement rod 141 with a mesh rod to an end of the mesh rod is greater than or equal to 1/5 of the length of the mesh rod, and the length of the reinforcement rod 141 is greater than or equal to 1/2 of the width of the most proximal mesh 12', so that the reinforcement rod 141 can provide a binding force to a large area of the most proximal mesh 12', so as to prevent reduced apposition of the oblique opening portion 11 caused by excessive stretching during thrombectomy. In addition, when reducing the radial dimension of the retrieval frame 10 for thrombectomy in a portion with a small inner diameter of a blood vessel, the reinforcement rod 141 can provide a binding force to a large area of the most proximal mesh 12', increasing the axial tensile strength of the first axial section A1 to reduce the difference in axial tensile strength between the first axial section A1 and the second axial section A2, making the radial reduction amplitudes of the first axial section A1 and the second axial section A2 closer to each other, reducing irritation of the first axial section A1 and the second axial section A2 to the blood vessel while keeping the oblique opening portion 11 apposed to the inner wall of the blood vessel.

In the embodiment shown in FIGS. 3 and 4, the most proximal mesh 12' is a quadrilateral mesh, and two reinforcement rods 141 are arranged in the most proximal mesh 12'. Two ends of each reinforcement rod 141 are respectively connected to two non-directly connected mesh rods of the quadrilateral mesh (i.e., one proximal mesh rod 113' and one distal mesh rod 121'), and the included angle α formed by each reinforcement rod 141 with the straight line L is ranged from 12° to 74°. A minimum distance from a connection point of each reinforcement rod 141 with a mesh rod (proximal mesh rod 113' or distal mesh rod 121') to an end of the mesh rod is greater than or equal to 1/5 of the length of the mesh rod, so that the two reinforcement rods 141 can apply a binding force to a large area of the most proximal mesh 12', and under the binding action of the two reinforcement rods 141, the structural stability of the most proximal mesh 12' is higher, which is more conducive to improving the axial tensile strength of the first axial section A1 and reducing the difference in axial tensile strength between the first axial section A1 and the second axial section A2. In addition, the two reinforcement rods 141 intersect and are connected at the intersection, which can further improve the structural stability of the most proximal mesh 12', helping increase the tensile strength of the most proximal mesh 12' and reduce the difference in axial tensile strength between the first axial section A1 and the second axial section A2.

Referring to FIG. 2, the pulling tube 20 is connected to the proximal end 112 of the oblique opening portion 11 via the collar 41 and the connecting strip 51. Therefore, when the pulling tube 20 applies a pulling force to the retrieval frame 10, the action point of the pulling force is the proximal end 112 of the oblique opening portion 11. Thus, under the same pulling force, the deformation amount of the first meshes 12 closer to the proximal end 112 in the circumferential and axial directions is larger. Therefore, under the same other conditions, the deformation amount of the most proximal mesh 12' is relatively larger than that of other first meshes 12 penetrated by the straight line L. Arranging the reinforcement rods 141 in all the first meshes 12 penetrated by the straight line L (including the most proximal mesh 12') is more conducive to increasing the axial tensile strength of the first axial section A1 and reducing the difference in axial tensile strength between the first axial section A1 and the second axial section A2.

In the embodiment shown in FIG. 3, the reinforcement rod 141 is arranged in the most proximal mesh 12' of the first axial section A1. Under the same other conditions (e.g., material of the reinforcement rod 141, rod width of the reinforcement rod 141, thickness of the reinforcement rod 141, magnitude of the included angle α formed by the reinforcement rod 141 with the straight line L, etc.), arranging the reinforcement rod 141 in the most proximal mesh 12' of the first axial section A1 is more conducive to improving the overall axial tensile strength of the first axial section A1, and more conducive to reducing the amplitude of axial stretching of the oblique opening portion 11 during dragging the retrieval frame 10 in the blood vessel by the pulling tube 20, thereby helping improve the apposition of the oblique opening portion 11. In addition, when axial tensile force is applied to both ends of the retrieval frame 10 to reduce the radial dimension of the retrieval frame 10, the higher the axial tensile strength of the first axial section A1, the more conducive to reducing the difference in axial tensile strength between the first axial section A1 and the second axial section A2. The retrieval frame 10 makes the radial dimension reduction amplitudes of the first axial section A1 and the second axial section A2 closer to each other, reducing irritation of the second axial section A2 to the blood vessel while keeping the oblique opening portion 11 apposed to the inner wall of the blood vessel.

Therefore, in an embodiment, the reinforcement rods 141 are arranged in both the most proximal mesh 12' and other first meshes 12 penetrated by the straight line L, and the thickness of the reinforcement rods 141 in the most proximal mesh 12' is greater than that of the reinforcement rods 141 in other first meshes 12. Thus, the ratio of axial tensile strength of the first axial section A1 to that of the second axial section A2 is set to ranged from 0.7 to 1, so that the difference in axial tensile strength between the first axial section A1 and the second axial section A2 is further reduced, thereby making the radial dimension reduction amplitudes of the first axial section A1 and the second axial section A2 closer to each other when adjusting the radial dimension of the retrieval frame 10, reducing irritation of the second axial section A2 to a portion with a small inner diameter of a blood vessel while keeping the oblique opening portion 11 apposed to the inner wall of the blood vessel.

### Second Embodiment

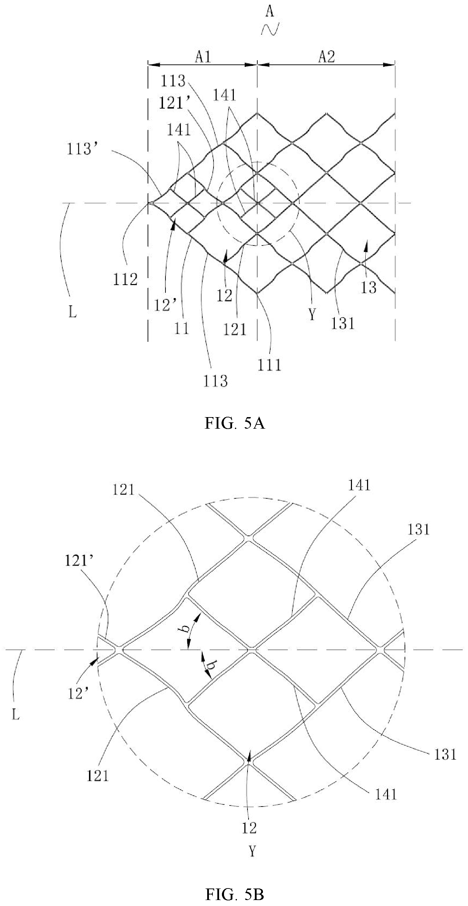

Referring to FIGS. 5A and 5B, in an embodiment, at least one first mesh 12 other than the most proximal mesh 12' in the axial section in which the oblique opening portion 11 is located is further provided with a reinforcement rod 141, and at least one reinforcement rod 141 is arranged in each first mesh 12 of the at least one first mesh 12. An included angle β formed by each reinforcement rod 141 with the straight line L is ranged from 12° to 74°, and a minimum distance from a connection point of each reinforcement rod 141 with the first mesh rod 121 to an end of the first mesh rod 121 is greater than or equal to 1/5 of the length of the first mesh rod 121, so that the reinforcement rod 141 has a long length and can provide a binding force to a large area of the first mesh 12 connected thereto, increasing the axial tensile strength of the first axial section A1 and reducing the difference in axial tensile strength between the first axial section A1 and the second axial section A2. So that, when the radial dimension of the retrieval frame 10 is operated to be reduced, the radial dimension contraction amplitudes of the first axial section A1 and the second axial section A2 are closer to each other, reducing irritation of the retrieval frame 10 on a portion with a small inner diameter of a blood vessel while keeping the oblique opening portion and the inner wall of the blood vessel in contact with each other. Meanwhile, the included angle β formed by each reinforcement rod 141 with the straight line L is ranged from 12° to 74°, and a minimum distance from a connection point of each reinforcement rod 141 with the first mesh rod 121 to an end of the first mesh rod 121 is greater than or equal to 1/5 of the length of the first mesh rod 121, so that the first mesh 12 connected to the reinforcement rod 141 can be compressed in the circumferential direction, facilitating loading of the retrieval frame 10 into the delivery sheath 30 with a small diameter and thus delivery in a body cavity of the patient.

The area covered by the most proximal mesh 12' has a tip structure pointing to the proximal end, i.e., the area covered by the most proximal mesh 12' is small. Even if α=0°, the reinforcement rod 141 is parallel to the straight line L, and after two ends of the reinforcement rod 141 are fixedly connected to the proximal mesh rod 113' and the distal mesh rod 121', the axial distance between connection points of the first mesh 12 fixedly connected to two ends of the reinforcement rod 141 is basically locked. In this case, the part of the first mesh 12 connected to the reinforcement rod 141 cannot be axially stretched or can only be stretched to a small extent under the action of pulling force, thereby causing the first mesh 12 fixedly connected to the reinforcement rod 141 to be unable to be radially compressed or only to be radially compressed to a small extent. However, the radial dimension of the area covered by the most proximal mesh 12' is still small, and the retrieval frame 10 can be loaded into the delivery sheath 30 with a small diameter for delivery in the body cavity of the patient.

Even if α=90°, the reinforcement rod 141 is perpendicular to the straight line L, and the width between connection points of the most proximal mesh 12' with two ends of the reinforcement rod 141 is basically locked. In this case, under the action of pulling force, the width between the two connection points of the most proximal mesh 12' connected to the reinforcement rod 141 cannot be reduced or can only be reduced to a small extent, thereby causing the most proximal mesh 12' fixedly connected to the reinforcement rod 141 to be unable to be radially compressed or only to be radially compressed to a small extent. However, the radial dimension of the area covered by the most proximal mesh 12' is still small, and the retrieval frame 10 can be loaded into the delivery sheath 30 with a small diameter for delivery in the body cavity of the patient.

However, the total area covered by all first meshes 12 other than the most proximal mesh 12' in the first axial section A1 is larger than that covered by the most proximal mesh 12'. The total area covered by all first meshes 12 other than the most proximal mesh 12' should be radially compressed as much as possible to reduce the insertion size.

Therefore, to balance tensile resistance and reduce the insertion size, when the reinforcement rods 141 are arranged in the first meshes 12 other than the most proximal mesh 12' in the first axial section A1, the included angle β formed by the reinforcement rod 141 with the straight line L is neither 0° nor 90°.

In this embodiment, the included angle β is ranged from 12° to 74°, so that after the reinforcement rods 141 are arranged in the first meshes 12 other than the most proximal mesh 12' in the first axial section A1, the first meshes 12 can still be radially compressed to a large extent for insertion into the delivery sheath 30. Therefore, even if at least one first mesh 12 other than the most proximal mesh 12' in the first axial section A1 is further provided with the reinforcement rod 141, the insertion size of the retrieval frame 10 will not be greatly increased. In addition, in this embodiment, a minimum distance from a connection point of each reinforcement rod 141 with the first mesh rod 121 to an end of the first mesh rod 121 is greater than or equal to 1/5 of the length of the first mesh rod 121, so that the reinforcement rod 141 has a long length, helping improve the axial tensile resistance of the first axial section A1 to reduce the difference in axial tensile strength between the first axial section A1 and the second axial section A2. Thus, the retrieval frame 10 has a small insertion size and can also prevent reduced apposition of the oblique opening portion 11 of the retrieval frame 10 caused by excessive stretching during thrombectomy. In addition, when the retrieval frame 10 enters a blood vessel with a small inner diameter, since the difference in axial tensile strength between the first axial section A1 and the second axial section A2 is small, the radial dimension reduction amplitudes of the first axial section A1 and the second axial section A2 after axial stretching are relatively close, so that irritation of the second axial section A2 to the blood vessel can be reduced while the oblique opening portion 11 is kept apposed to the blood vessel wall.

It should be noted that when a part of the first mesh 12 in which the reinforcement rod 141 is located is in the first axial section A1 and another part is in the second axial section A2, it also falls within the scope defined by "the reinforcement rod 141 is located in the first mesh 12 of the first axial section A1". In this embodiment, at least a part of the first axial section A1 (i.e., the first mesh fixedly connected to the reinforcement rod 141) can be constrained by the reinforcement rod 141. During applying a pulling force to the pulling tube 20 to drive the retrieval frame 10 to move proximally, the reinforcement rod 141 can reduce the amplitude of axial stretching of the first axial section A1, thereby improving the apposition of the oblique opening portion 11. In addition, when axial tensile force is applied to both ends of the retrieval frame 10 via the pulling tube 20 and the inner tube 60, the radial contraction amplitudes of the first axial section A1 and the second axial section A2 are closer to each other, so as to balance reducing irritation of the retrieval frame 10 to a blood vessel with a small inner diameter and keeping the oblique opening portion 11 apposed to the inner wall of the blood vessel.

In the embodiment shown in FIGS. 5A and 5B, each first mesh 12 (including the most proximal mesh 12') where the reinforcement rod 141 is located is penetrated by the straight line L. Since the force applied to the pulling tube 20 is axial tensile force, i.e., the force applied to the proximal end 112 is axial tensile force, and the straight line L extends axially, arranging the reinforcement rod 141 in the first mesh 12 penetrated by the straight line L can better resist axial tensile force, so as to increase the axial tensile strength of the first axial section A1 and reduce the difference in axial tensile strength between the first axial section A1 and the second axial section A2.

In other embodiments, even if only a part of the first meshes 12 where the reinforcement rods 141 are located is penetrated by the straight line L, the overall axial tensile strength of the first axial section A1 can be improved and the difference in axial tensile strength between the first axial section A1 and the second axial section A2 can be reduced.

### Third Embodiment

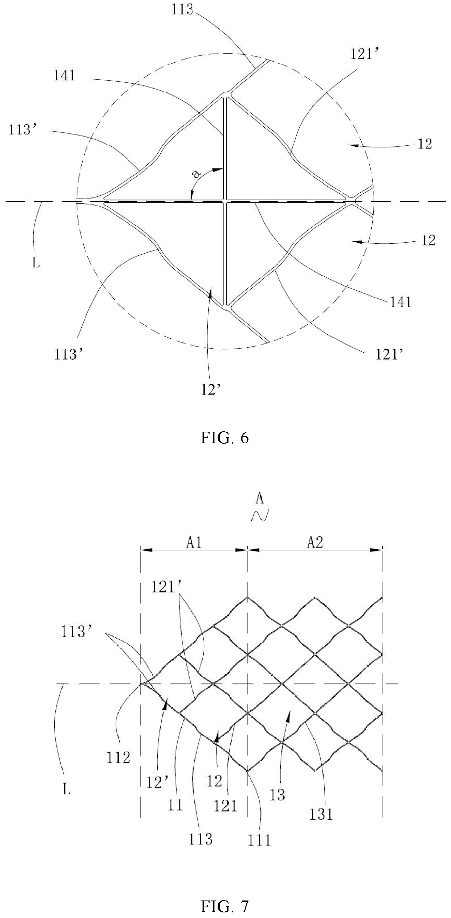

Referring to FIG. 6, the third embodiment differs from the first embodiment in that the most proximal mesh 12' is a quadrilateral mesh, and two reinforcement rods 141 are arranged in the most proximal mesh 12'. Two ends of one reinforcement rod 141 are respectively connected to two vertices of the quadrilateral mesh, and two ends of the other reinforcement rod 141 are respectively connected to the other two vertices of the quadrilateral mesh. In addition, the included angle α formed by one reinforcement rod 141 with the straight line L is 0°, the included angle α formed by the other reinforcement rod 141 with the straight line L is 90°, the two reinforcement rods 141 intersect, and the two reinforcement rods 141 are connected at the intersection. Thus, during thrombectomy, when the retrieval frame 10 moves under the action of pulling force, the most proximal mesh 12' cannot be axially stretched or can only be stretched to a small extent, and cannot be circumferentially compressed or can only be compressed to a small extent, so that the structural stability of the most proximal mesh 12' is greatly improved under the combined action of the two reinforcement rods 141. The deformation amount of the most proximal mesh 12' in the axial and radial directions under the action of pulling force is very small or zero, thereby greatly improving the axial tensile strength of the first axial section A1 and greatly reducing the difference between the first axial section A1 and the second axial section A2. This reduces or prevents reduced apposition of the oblique opening portion 11 caused by pulling force during thrombectomy, and keeps the oblique opening portion 11 apposed to the inner wall of the blood vessel while reducing the outer diameter of the retrieval frame 10 to reduce irritation of the retrieval frame 10 to a blood vessel with a small inner diameter. In addition, since the two reinforcement rods 141 are connected at the intersection, the structural stability of the most proximal mesh 12' constrained by the two reinforcement rods 141 can be further improved, further increasing the axial tensile strength of the first axial section A1.

### Fouth Embodiment

Referring to FIG. 7, in this embodiment, the rod width of mesh rods (including two proximal mesh rods 113' and two distal mesh rods 121') of the most proximal mesh 12' is greater than that of at least part of second mesh rods 131 of second meshes 13 in the second axial section A2, so that a ratio of a total volume of the two edges 113 and the plurality of first mesh rods 121 of the first axial section A1 to a total volume of the plurality of second mesh rods 131 of the second axial section A2 is ranged from 0.65 to 1. This increases the axial tensile strength of the first axial section A1 and reduces the difference in axial tensile strength between the first axial section A1 and the second axial section A2, making the ratio of axial tensile strength of the first axial section A1 to that of the second axial section A2 ranged from 0.65 to 1, reducing or preventing reduced apposition of the oblique opening portion 11 caused by pulling force during thrombectomy. When the retrieval frame 10 performs thrombectomy in a portion with a small inner diameter of a blood vessel, irritation of the second axial section A2 to the inner wall of the blood vessel can be reduced while the oblique opening portion 11 is kept apposed to the inner wall of the blood vessel.

In an embodiment, the rod width of the oblique opening portion 11 is greater than that of at least part of mesh rods 131 of second meshes 13 in the second axial section A2, so as to increase the axial tensile strength of the first axial section A1 and reduce the difference in axial tensile strength between the first axial section A1 and the second axial section A2.

### Fifth Embodiment

This embodiment differs from the fourth embodiment in that the rod width of first mesh rods 121 of part of first meshes 12 in the plurality of first meshes 12 other than the most proximal mesh 12' in the first axial section A1 is greater than that of at least part of second mesh rods 131 of second meshes 13 in the second axial section A2, so as to increase the axial tensile strength of the first axial section A1 and reduce the difference in axial tensile strength between the first axial section A1 and the second axial section A2. The part of first meshes 12 in the first axial section A1 is arranged on the same axis as the most proximal mesh 12', and the part of first meshes 12 in the first axial section A1 is penetrated by the straight line L passing through the proximal end of the oblique opening portion 11 and the distal end of the retrieval frame 10, which is more conducive to increasing the axial tensile strength of the first axial section A1 and reducing the difference in axial tensile strength between the first axial section A1 and the second axial section A2.

The technical features of the above embodiments can be combined arbitrarily. To simplify the description, all possible combinations of the technical features in the above embodiments are not described. However, as long as there is no contradiction in the combination of these technical features, they shall be considered to be within the scope recorded in this specification.

The above disclosures are merely preferred embodiments of the present invention, which certainly cannot be used to limit the patent scope of the present invention. Therefore, equivalent changes made according to the claims of the present invention still fall within the scope covered by the present invention.

## Claims

1. A thrombectomy apparatus, **characterized by** comprising:
a retrieval frame comprising a proximal support and a distal support connected to a distal end of the proximal support, the retrieval frame having an inner cavity extending through the proximal support and the distal support, the proximal support comprising a first axial section and a second axial section connected to a distal end of the first axial section, a proximal end of the first axial section being provided with an oblique opening portion communicating with the inner cavity, the first axial section having a tip structure pointing toward a proximal end, the second axial section having a circumferential outer contour, and a ratio of axial tensile strength of the first axial section to axial tensile strength of the second axial section being ranged from 0.6 to 1;
a pulling tube connected to the tip structure; and
an inner tube slidably disposed through the pulling tube, a distal end of the inner tube being connected to a distal end of the distal support, and the retrieval frame being radially shrinkable in response to a sliding of the inner tube distally relative to the pulling tube.

2. The thrombectomy apparatus according to claim 1, **characterized in that** the oblique opening portion has a proximal end and a distal end, and when the retrieval frame is planarly expanded along a straight line, as a cutting line, from the distal end of the oblique opening portion to the distal end of the distal support: the first axial section is a first mesh structure with a triangular outer contour, the proximal end is one vertex of the triangle, and two edges of the triangle extending distally from the vertex form the oblique opening portion in a closed state; and the first mesh structure comprises a plurality of first meshes defined by the two edges and a plurality of first mesh rods.

3. The thrombectomy apparatus according to claim 2, **characterized in that** when the retrieval frame is planarly expanded along a straight line, as a cutting line, from the distal end of the oblique opening portion to the distal end of the distal support: the second axial section is a second mesh structure defined by two radially opposed wavy lines, proximal ends of the two wavy lines are respectively connected to distal ends of the two edges, a maximum radial distance between the two wavy lines is equal to a radial distance between the distal ends of the two edges, and the second mesh structure comprises a plurality of second meshes defined by a plurality of second mesh rods; and
a reinforcement structure is arranged in the first mesh structure, and a ratio of a total volume of the two edges, the plurality of first mesh rods and the reinforcement structure of the first axial section to a total volume of the plurality of second mesh rods of the second axial section ranges from 0.65 to 1.

4. The thrombectomy apparatus according to claim 3, **characterized in that** the plurality of first meshes comprises a most proximal mesh, the most proximal mesh is defined by parts of the two edges and two first mesh rods, a proximal vertex of the most proximal mesh is the vertex of the triangle, the reinforcement structure comprises at least one reinforcement rod arranged in the most proximal mesh, two ends of each reinforcement rod are respectively fixedly connected to two of the two first mesh rods and the two edges of the most proximal mesh; when the retrieval frame is planarly expanded along a straight line, as a cutting line, from the distal end of the oblique opening portion to the distal end of the distal support: the at least one reinforcement rod forms an included angle α with a straight line passing through the proximal end of the oblique opening portion and the distal end of the distal support, and the included angle α ranges from 0° to 90°; when the included angle α ranges from 0° to less than 12°, and a length of the reinforcement rod is greater than or equal to 1/2 of an axial length of the most proximal mesh; when the included angle α ranges from 12° to 74°, and a minimum distance from a connection point of each reinforcement rod with two of the two first mesh rods and the two edges to ends of the two first mesh rods and the two edges is greater than or equal to 1/5 of a length of the first mesh rod; and when the included angle α ranges from more than 74° to 90°, a minimum distance from a connection point of each reinforcement rod with two of the two first mesh rods and the two edges to ends of the two first mesh rods and the two edges is greater than or equal to 1/5 of a length of the first mesh rod or the edge where it is located, and a length of the reinforcement rod is greater than or equal to 1/2 of a width of the most proximal mesh.

5. The thrombectomy apparatus according to claim 4, **characterized in that** the reinforcement structure further comprises a reinforcement rod arranged in at least one first mesh other than the most proximal mesh in the first axial section, at least one reinforcement rod is arranged in each of the at least one first mesh other than the most proximal mesh, and each reinforcement rod outside the most proximal mesh is connected to two of the first mesh rods of the first mesh where it is located; and when the retrieval frame is planarly expanded along a straight line, as a cutting line, from the distal end of the oblique opening portion to the distal end of the distal support: each reinforcement rod outside the most proximal mesh forms an included angle β with a straight line passing through the proximal end of the oblique opening portion and the distal end of the retrieval frame, the included angle β ranges from 12° to 74°, and a minimum distance from a connection point of each reinforcement rod outside the most proximal mesh with the first mesh rod to an end of the first mesh rod is greater than or equal to 1/5 of a length of the first mesh rod.

6. The thrombectomy apparatus according to claim 5, **characterized in that** the reinforcement rods are arranged in part of the first meshes of the plurality of first meshes other than the most proximal mesh in the first axial section, the part of the first meshes are arranged on the same axis as the most proximal mesh, and the part of the first meshes are penetrated by the straight line.

7. The thrombectomy apparatus according to claim 4, **characterized in that** the most proximal mesh is a quadrilateral mesh, two reinforcement rods are arranged in the most proximal mesh, and two ends of each of the two reinforcement rod are respectively connected to the first mesh rods and the edges; each reinforcement rod forms an included angle α ranging from 12° to 74° with the straight line; a minimum distance from a connection point of each reinforcement rod with two of the two first mesh rods and the two edges to ends of the two first mesh rods and the two edges is greater than or equal to 1/5 of a length of the first mesh rod or the edge where it is located; and the two reinforcement rods intersect and are connected at an intersection; or
the most proximal mesh is a quadrilateral mesh, two reinforcement rods are arranged in the most proximal mesh, two ends of one reinforcement rod are respectively connected to two vertices of the quadrilateral mesh, two ends of the other reinforcement rod are respectively connected to the other two vertices of the quadrilateral mesh; one reinforcement rod forms an included angle α of 0° with the straight line, and the other reinforcement rod forms an included angle α of 90° with the straight line; and the two reinforcement rods intersect and are connected at an intersection.

8. The thrombectomy apparatus according to claim 2, **characterized in that** when the retrieval frame is planarly expanded along a straight line, as a cutting line, from the distal end of the oblique opening portion to the distal end of the distal support: the second axial section is a second mesh structure defined by two radially opposed wavy lines, proximal ends of the two wavy lines are respectively connected to distal ends of the two edges, a maximum radial distance between the two wavy lines is equal to a radial distance between the distal ends of the two edges, and the second mesh structure comprises a plurality of second meshes defined by a plurality of second mesh rods; and
the plurality of first meshes comprises a most proximal mesh, the most proximal mesh is defined by parts of the two edges and two first mesh rods, and a proximal vertex of the most proximal mesh is the vertex of the triangle; a rod width of the first mesh rods of the most proximal mesh is greater than a rod width of second mesh rods of at least part of second meshes in the second axial section, and a rod width of parts of the edges for forming the most proximal mesh is greater than a rod width of second mesh rods of at least part of second meshes in the second axial section; and a ratio of a total volume of the two edges and the plurality of first mesh rods of the first axial section to a total volume of the plurality of second mesh rods of the second axial section ranges from 0.65 to 1.

9. The thrombectomy apparatus according to claim 8, **characterized in that** a rod width of first mesh rods of part of first meshes of the plurality of first meshes other than the most proximal mesh in the first axial section is greater than a rod width of second mesh rods of at least part of second meshes in the second axial section, the part of first meshes are arranged on the same axis as the most proximal mesh, and the part of first meshes in the first axial section are penetrated by a straight line passing through the proximal end of the oblique opening portion and the distal end of the retrieval frame.

10. The thrombectomy apparatus according to claim 8 or 9, **characterized in that** a reinforcement structure is arranged in the first mesh structure, the reinforcement structure comprises at least one reinforcement rod arranged in the most proximal mesh, and two ends of each reinforcement rod are respectively fixedly connected to two of the two first mesh rods and the two edges of the most proximal mesh; when the retrieval frame is planarly expanded along a straight line, as a cutting line, from the distal end of the oblique opening portion to the distal end of the distal support: the at least one reinforcement rod forms an included angle α with a straight line passing through the proximal end of the oblique opening portion and the distal end of the distal support, the included angle α ranges from 0° to 90°; when the included angle α ranges from 0° to less than 12°, a length of the reinforcement rod is greater than or equal to 1/2 of an axial length of the most proximal mesh; when the included angle α ranges from 12° to 74°, a minimum distance from a connection point of each reinforcement rod with two of the two first mesh rods and the two edges to ends of the two first mesh rods and the two edges is greater than or equal to 1/5 of a length of the first mesh rod or the edge where it is located; and when the included angle α ranges from more than 74° to 90°, a minimum distance from a connection point of each reinforcement rod with two of the two first mesh rods and the two edges to ends of the two first mesh rods and the two edges is greater than or equal to 1/5 of a length of the first mesh rod, and a length of the reinforcement rod is greater than or equal to 1/2 of a width of the most proximal mesh.

11. The thrombectomy apparatus according to claim 10, **characterized in that** the reinforcement structure further comprises reinforcement rods arranged in at least one first mesh other than the most proximal mesh in the first axial section, and at least one reinforcement rod is arranged in each of the at least one first mesh other than the most proximal mesh, each reinforcement rod outside the most proximal mesh is connected to two first mesh rods of the first mesh where it is located; when the retrieval frame is planarly expanded along a straight line, as a cutting line, from the distal end of the oblique opening portion to the distal end of the distal support: each reinforcement rod outside the most proximal mesh forms an included angle β with a straight line passing through the proximal end of the oblique opening portion and the distal end of the retrieval frame, the included angle β ranges from 12° to 74°; and a minimum distance from a connection point of each reinforcement rod outside the most proximal mesh with the first mesh rod to an end of the first mesh rod is greater than or equal to 1/5 of a length of the first mesh rod.

12. The thrombectomy apparatus according to claim 8, **characterized in that** a rod width of the edges is greater than a rod width of second mesh rods of at least part of second meshes in the second axial section.
